# EUROPEAN PATENT APPLICATION

(11) **EP 3 949 875 A1**
(43) Date of publication of application: **09.02.2022**
(21) Application number: 20779347.2
(22) Date of filing: 20.03.2020
(51) Int. Cl.: A61B 17/12, A61B 17/122

(54) **HYDRAULICALLY DRIVEN CLIP APPLIER**

(30) Priority: 28.03.2019 JP 2019063569
(71) Applicant: Kawasaki Jukogyo Kabushiki Kaisha, Hyogo 650-8670 (JP)
(72) Inventor: TANAKA, Hideki, Kobe-shi, Hyogo 6508670 (JP); ANADA, Tadashi, Kobe-shi, Hyogo 6508670 (JP); HOMMA, Toshiyuki, Kobe-shi, Hyogo 6508670 (JP)
(74) Representative: EIP
(86) International application number: PCT/JP2020/012564
(87) International publication number: WO 2020/196351

(57) **Abstract**

A hydraulically driven clip applier includes: a pair of jaws that respectively include, at a tip end, a pair of gripping portions capable of holding a clip therebetween and are coupled to each other in a manner to allow the pair of gripping portions to be opened and closed and when a base end of the pair of jaws is opened, close the pair of gripping portions; and an opening/closing driving mechanism that is a cylinder mechanism including a rod and when supplied with an operating fluid, extends the rod. Base end portions of the pair of the jaws are positioned at a distance from each other. The opening/closing driving mechanism is configured to position a tip end of the rod between the base end portions of the pair of jaws and open the base end of the pair of jaws by extending the rod.

## Description

### Technical Field

The present invention relates to hydraulically driven clip appliers for ligating tubular tissues, blood vessels, or the like in surgery.

### Background Art

In surgery assisted by a robot, a surgical procedure is performed by inserting an instrument attached to the tip of a robot arm into a patient's body, and various instruments for various uses can be attached to the tip of the robot arm. As such a surgical assist robot system, the surgical system disclosed in Patent Literature (PTL 1) is known, for example, and examples of the instrument to be attached include a clip applier. The clip applier is medical equipment for ligating a tubular tissue or a blood vessel, and is used to ligate a tubular tissue or a blood vessel by closing a clip in a ligating position.

### Citation List

### Patent Literature

PTL 1: Japanese Laid-Open Patent Application Publication No. 2018-020004

### Summary of Invention

### Technical Problem

Examples of a clip applier used in the surgical system disclosed in PTL 1 or the like include the following. Specifically, the clip applier includes: a driving device provided at the tip of the robot arm; and a pair of jaws configured to hold a clip between the jaws, and the driving device and the pair of jaws are connected by a hollow shaft. Furthermore, the clip applier includes, for example, a rod in order to transmit the force of the driving device to the pair of jaws. The rod is connected to the pair of jaws via a link, a cam, and the like. The rod can be pushed and pulled using the driving device; as a result of the rod being pushed and pulled, the pair of jaws is opened and closed.

In the clip applier having such a function, a rigid rod is used in order to efficiently transmit the pushing and pulling force to the pair of jaws, and the shaft into which the rod is inserted is also formed from a straight tube. Therefore, the shaft cannot be bent, meaning that the pair of jaws cannot reach some parts in a patient's body. In view of this, a clip applier of the wire drive type in which a wire is used instead of the rod has been proposed.

The clip applier of the wire drive type uses a wire to connect a driving mechanism and a pair of jaws and pushes and pulls the pair of jaws by the wire to open and close the pair of jaws. Therefore, the shaft can be made bendable, and the pair of jaws can be given better access in a patient's body. On the other hand, making the shaft bendable requires the wire to be also bent according to the condition of the bent shaft; in order to bend the wire, the wire needs to pass through more than one pulley. Therefore, at the time of driving, the friction between the pulley and the wire causes a reduction in driving force that is transmitted to the pair of jaws, making it difficult to obtain great clamping force. This leads to a limitation on the form of clips that can be used.

Thus, the present invention has an object to provide a clip applier capable of generating great clamping force.

### Solution to Problem

A hydraulically driven clip applier according to the first invention includes: a pair of jaws that respectively include, at a tip end, a pair of gripping portions capable of holding a clip between the gripping portions and are coupled to each other in a manner to allow the pair of the gripping portions to be opened and closed and when a base end of the pair of the jaws is opened, close the pair of the gripping portions; and an opening/closing driving mechanism that is a cylinder mechanism including a rod and when supplied with an operating fluid, extends the rod. Base end portions of the pair of the jaws are positioned at a distance from each other. The opening/closing driving mechanism is configured to position a tip end of the rod between the base end portions of the pair of the jaws and open the base end of the pair of the jaws by extending the rod.

According to the present invention, the pair of gripping portions is closed using the operating fluid, and thus it is possible to efficiently transmit force. Therefore, as compared to a conventional clip applier of the wire drive type, the gripping force can be made great; for example, it is possible to squeeze even a clip that requires great gripping force to squeeze, such as a metal clip.

In the above-described invention, it is preferable that the hydraulically driven clip applier further include a first biasing member that biases at least one of the pair of the jaws to operate the pair of the gripping portions in a direction in which the pair of the gripping portions is opened, the pair of the jaws be configured to be closed at the base end when the pair of the gripping portions is opened, and the opening/closing driving mechanism include a second biasing member that biases the rod against a pressure of the operating fluid to retract the rod.

According to this configuration, a single-acting cylinder mechanism can be used. Therefore, the structure of the clip applier can be simplified, and the number of components can be reduced. Thus, it is possible to reduce cost for clip appliers.

A hydraulically driven clip applier according to the second invention includes: a pair of jaws that respectively include, at a tip end, a pair of gripping portions capable of holding a clip between the gripping portions and are coupled to each other in a manner to allow the pair of the gripping portions to be opened and closed and when a base end of the pair of the jaws is opened, close the pair of the gripping portions; an opening/closing mechanism coupled to the base end of the pair of the jaws and configured to open and close the base end of the pair of the jaws; and a driving mechanism that operates a link of the opening/closing mechanism to open the base end of the pair of the jaws. It is preferable that the driving mechanism be a cylinder mechanism including a rod and when supplied with an operating fluid, retract the rod, and the opening/closing mechanism include a pair of links coupled to the rod and the base end of the pair of the jaws and be configured to open the base end of the pair of the jaws using the pair of the links when the rod is retracted.

According to this configuration, the pair of gripping portions is closed using the operating fluid, and thus it is possible to efficiently transmit force. Therefore, as compared to a conventional clip applier of the wire drive type, the gripping force can be made great; for example, it is possible to squeeze even a clip that requires great gripping force to squeeze, such as a metal clip.

In the above-described invention, it is preferable that the pair of the jaws open the pair of the gripping portions by closing the base end of the pair of the jaws, the opening/closing mechanism be configured to close the base end of the pair of the jaws using the pair of the links when the rod is extended, and the driving mechanism further include a biasing member that biases the rod against a pressure of the operating fluid to extend the rod.

According to this configuration, a single-acting cylinder mechanism can be used. Therefore, the structure of the clip applier can be simplified, and the number of components can be reduced. Thus, it is possible to reduce cost for clip appliers.

In the above-described invention, it is preferable that the cylinder mechanism include a cylinder through which the rod protruding from a tip end of the cylinder is inserted in a manner to be retractable, the cylinder include, at the tip end, a cylinder chamber to which the operating fluid is supplied to retract the rod, a tube through which the operating fluid is supplied be connected to a base end of the rod, and a communication passage be formed in the rod to guide, to the cylinder chamber, the operating fluid supplied through the tube.

According to this configuration, it is possible to reduce the increase in the diameter of the cylinder that is caused by forming, in the cylinder, a passage connecting the tube and the cylinder chamber. Thus, the increase in the size of the driving mechanism and the increase in the size of the clip applier can be reduced.

A hydraulically driven clip applier according to the third invention includes: a pair of jaws that respectively include, at a tip end, a pair of gripping portions capable of holding a clip between the gripping portions and are rotatably coupled to each other in a manner to allow the pair of the gripping portions to be opened along with a closing motion at a base end and allow the pair of the gripping portions to be closed along with an opening motion at the base end; an opening/closing mechanism coupled to the base end of the pair of the jaws and configured to open and close the base end of the pair of the jaws; and an opening/closing driving mechanism that, when supplied with an operating fluid, operates the opening/closing mechanism to open the base end of the pair of the jaws. It is preferable that the opening/closing driving mechanism be a cylinder mechanism including a rod and when supplied with the operating fluid, operates the rod, and the opening/closing mechanism include a pair of links coupled to the rod and the base end of the pair of the jaws and be configured to close the base end of the pair of the jaws using the pair of the links when the rod is operated.

According to this configuration, the pair of gripping portions is closed using the operating fluid, and thus it is possible to efficiently transmit force. Therefore, as compared to a conventional clip applier of the wire drive type, the gripping force can be made great; for example, it is possible to squeeze even a clip that requires great gripping force to squeeze, such as a metal clip.

### Advantageous Effects of Invention

According to the present invention, it is possible to generate great clamping force.

The above object, other objects, features, and advantages of the present invention will be made clear by the following detailed explanation of preferred embodiments with reference to the attached drawings.

### Brief Description of Drawings

Fig. 1 is a diagram illustrating the overview of a clip applier system including a clip applier according to Embodiment 1.
Fig. 2 is a plan cross-sectional view illustrating a cut casing portion of the clip applier illustrated in Fig. 1.
Fig. 3 is an exploded perspective view illustrating a pair of disassembled jaws in the clip applier illustrated in Fig. 1.
Fig. 4 is a diagram illustrating a pair of jaws closed in the clip applier illustrated in Fig. 1.
Fig. 5 is a plan cross-sectional view illustrating a clip applier according to Embodiment 2.
Fig. 6 is a side cross-sectional view illustrating the clip applier of Fig. 5 cut along cutting-plane line VI-VI.
Fig. 7 is a diagram illustrating a pair of jaws closed in the clip applier illustrated in Fig. 5.

### Description of Embodiments

With reference to the aforementioned drawings, the following describes hydraulically driven clip appliers (hereinafter referred to simply as "clip appliers") 1, 1A according to embodiments of the present invention. Note that the concept of directions mentioned in the following description is used for the sake of explanation and is not intended to limit the orientations, etc., of elements according to the present invention to these directions. The clip appliers 1, 1A described below are merely one embodiment of the present invention. Thus, the present invention is not limited to the embodiments and may be subject to addition, deletion, and alteration within the scope of the essence of the present invention. In the embodiments of the present invention, an area at the tip end of the clip appliers 1, 1A will be referred to as the "distal end/side", and an area at the base end of the clip appliers 1, 1A will be referred to as the "proximal end/side".

### Embodiment 1

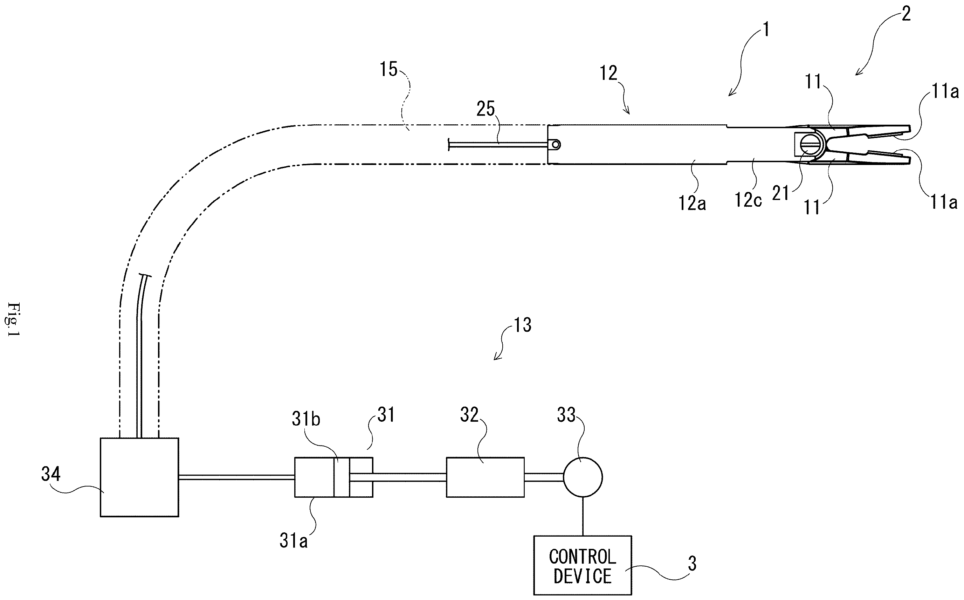

A clip applier system 2 illustrated in Fig. 1 is used to ligate a tubular tissue, a blood vessel, or the like (hereinafter referred to as a "blood vessel, etc.") in various surgical operations assisted by robots. The clip applier system 2 includes a clip applier 1 and a control device 3, and a tubular tissue, a blood vessel, or the like (hereinafter referred to as a "blood vessel, etc.") of a patient is ligated using the clip applier 1. The clip applier 1 is configured as follows.

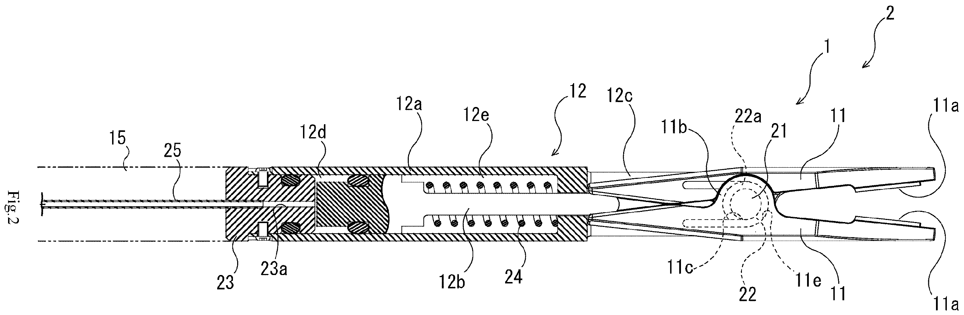

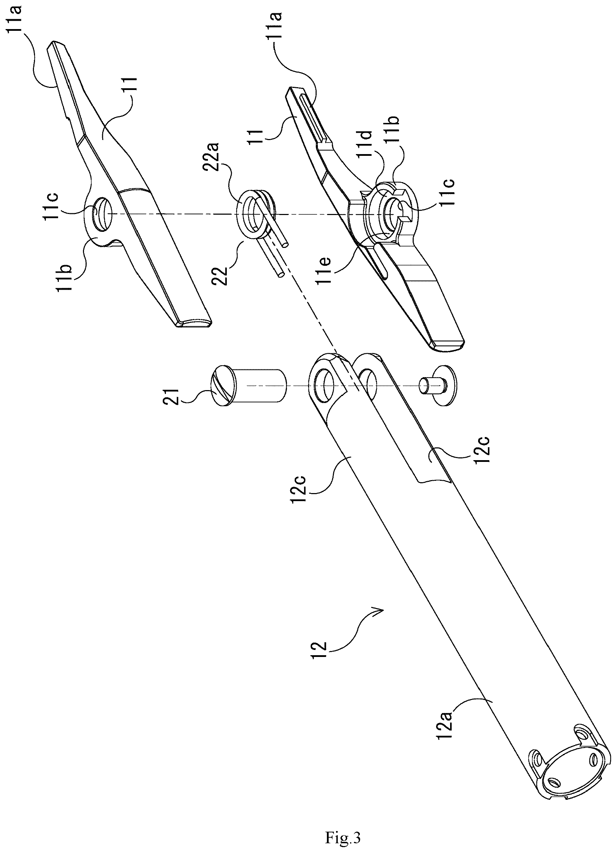

The clip applier 1 includes a pair of jaws 11, 11, an opening/closing driving mechanism 12, and a driving device 13. The pair of jaws 11, 11 includes, at the tip ends thereof, gripping portions 11a, 11a, respectively, as illustrated in Figs. 2 and 3, and is configured to be opened and closed so that the pair of gripping portions 11a, 11a are brought close to each other and separated from each other. More specifically, the pair of jaws 11, 11 are elongated members and are arranged close to each other so that one side surface of one of the jaws 11, 11 and one side surface of the other of the jaws 11, 11 face each other. On side surfaces of the pair of jaws 11, 11 that are located on one side at the tip end thereof, the gripping portions 11a, 11a are formed so as to face each other, and a clip not illustrated in the drawings can be loaded between the gripping portions 11a, 11a. Furthermore, the pair of jaws 11, 11 includes coupling portions 11b, 11b in the middle in the longitudinal direction.

The coupling portions 11b, 11b protrude laterally outward from the remaining portions and are each formed in the shape of a circle as seen in plan view. The height of the coupling portions 11b, 11b is set to half the height of the remaining portions of the pair of jaws 11, 11, and the coupling portions 11b, 11b overlap each other. The coupling portions 11b, 11b have through-holes 11c, 11c, respectively, at the center, and a pin member 21 is inserted through the through-holes 11c, 11c.

The pair of jaws 11, 11 configured as described above are coupled to each other by the pin member 21 and can rotate about the pin member 21. Specifically, by rotating the pair of jaws 11, 11, it is possible to move tip portions of the pair of jaws 11, 11 toward and away from each other (in other words, it is possible to open and close the tip portions (specifically, the pair of gripping portions 11a, 11a) of the pair of jaws 11, 11). Furthermore, recessed portions 11d, 11d are formed in facing surfaces of the coupling portions 11b, 11b, and a spring housing space 11e is formed by the recessed portions 11d, 11d fitting with each other. A torsion coil spring 22 is housed in the spring housing space 11e.

More specifically, the torsion coil spring 22 which is one example of the first biasing member is housed in the spring housing space 11e with a coil portion 22a of the torsion coil spring 22 attached to the exterior of the pin member 21. Furthermore, the torsion coil spring 22 has one end portion fixed to one jaw 11 and the other end portion fixed to the other jaw 11. The torsion coil spring 22 positioned in this manner biases at least one of the pair of jaws 11, 11 so as to open the pair of gripping portions 11a, 11a, in other words, move base ends of the gripping portions 11a, 11a toward each other. Meanwhile, the opening/closing driving mechanism 12 is provided on the pair of jaws 11, 11 in order to move the base ends of the pair of jaws 11, 11 away from each other to close the pair of gripping portions 11a, 11a.

The opening/closing driving mechanism 12 is what is called a cylinder mechanism and includes a cylinder 12a and a rod 12b, as illustrated in Fig. 2. The cylinder 12a is formed in the approximate shape of a pipe with a closed end and has a bottom at the tip end. Furthermore, a pair of attachment pieces 12c, 12c is integrally formed on a tip portion of the cylinder 12a (refer to Fig. 3). The pair of attachment pieces 12c, 12c are each a member in the approximate shape of a plate and are arranged so that one surface of one attachment piece 12c and one surface of the other attachment piece 12c face each other. The base end of the pair of jaws 11, 11 is inserted between the pair of attachment pieces 12c, 12c arranged as just described.
Furthermore, the pin member 21 is inserted through the pair of attachment pieces 12c, 12c, and the pair of jaws 11, 11 are rotatably fastened to the pair of attachment pieces 12c, 12c by the pin member 21. In this manner, the pair of jaws 11, 11 is attached to the cylinder 12a via the pair of attachment pieces 12c, 12c. Furthermore, the cylinder 12a has an opening portion covered by a lid body 23, as illustrated in Fig. 2, and the rod 12b is housed in this covered space so as to be movable to open and close the pair of jaws 11, 11.

A tip end portion of the rod 12b penetrates the tip end of the cylinder 12a and protrudes from the tip end of the cylinder 12a toward the pair of jaws 11, 11. The tip of the rod 12b is formed in the approximate shape of a partial sphere and abuts one side surface of each of the pair of jaws 11, 11 that is located at the base end thereof. The base end of the pair of jaws 11, 11 is formed so that the distance between the facing side surfaces thereof increases toward the base end (in other words, towards the proximal end). In other words, the inner surface of the base end of the pair of jaws 11, 11 is tapered in shape. Therefore, as the rod 12b is pushed forward with the tip abutting the inner surface, the base end of the pair of jaws 11, 11 is opened, and thus the tip end of the pair of jaws 11, 11 is closed.

The rod 12b is formed so that an middle portion thereof is larger in diameter than the remaining portion, and the outer diameter of the rod 12b substantially matches the inner diameter of the cylinder 12a. Therefore, the inner space of the cylinder 12a is divided into two spaces; the space located closer to the opening of the cylinder 12a than the middle portion forms a cylinder chamber 12d. The cylinder chamber 12d is a space formed between a base end portion of the rod 12b and the lid body 23. Furthermore, a tube 25 is connected to the lid body 23 in order to supply the operating fluid (for example, saline or oil) to the cylinder chamber 12d. The tube 25 is connected to the driving device 13 as described in detail later, and is connected to the cylinder chamber 12d via a communication passage 23a formed in the lid body 23. Therefore, the driving device 13 supplies the operating fluid to the cylinder chamber 12d via the tube 25 and the communication passage 23a and removes the operating fluid from the cylinder chamber 12d via the tube 25 and the communication passage 23a, for example. The rod 12b receives, by the base end portion, the pressure of the operating fluid located in the cylinder chamber 12d, and when the operating fluid is supplied to the cylinder chamber 12d, the rod 12b is extended.
Thus, the base end of the pair of jaws 11, 11 is opened, and the tip end of the pair of jaws 11, 11 is closed accordingly.

Inside the cylinder 12a, a space located closer to the bottom end than the middle portion of the rod 12b forms a spring housing space 12e. A compression coil spring 24 is housed in the spring housing space 12e. The compression coil spring 24 which is one example of the second biasing member is disposed in the spring housing space 12e with the rod 12b inserted through the compression coil spring 24, and biases the rod 12b against the pressure of the operating fluid in the cylinder chamber 12d. Therefore, when the driving device 13 removes the operating fluid from the cylinder chamber 12d, the rod 12b is retracted with assistance of the biasing force of the compression coil spring 24, and the tip of the rod 12b moves away from the pair of jaws 11, 11 (in other words, toward the proximal end). Accordingly, the torsion coil spring 22 causes the base end of the pair of jaws 11, 11 to be closed, opening the tip end of the pair of jaws 11, 11.

In this manner, the opening/closing driving mechanism 12 can open and close the tip end of the pair of jaws 11, 11 by supplying and removing the operating fluid, and the driving device 13 is connected to the opening/closing driving mechanism 12 via the tube 25 in order to cause the pair of jaws 11, 11 to perform the opening/closing motion. The driving device 13 includes a pump 31, a direct-acting mechanism 32, and a motor 33. The pump 31, which is, for example, a piston pump, is connected to the cylinder chamber 12d of the opening/closing driving mechanism 12 via the tube 25. More specifically, the pump 31 includes a cylinder 31a and a piston 31b, and when the piston 31b is retracted (in other words, pushed down), the operating fluid is supplied from the cylinder 31a to the cylinder chamber 12d via the tube 25. When the piston 31b is extended (in other words, pulled up), the operating fluid is removed from the cylinder chamber 12d into the cylinder 31a via the tube 25. Furthermore, the motor 33 is provided on the pump 31 via the direct-acting mechanism 32 in order to extend and retract the piston 31b.

The motor 33, which is, for example, a servomotor, can rotate a shaft member (not illustrated in the drawings) to be described later, both forward and backward, and rotates the shaft member with torque corresponding to an electric current flowing thereto. The direct-acting mechanism 32 is provided on the motor 33 having such a function. The direct-acting mechanism 32 converts a rotational motion into a linear motion; for example, the direct-acting mechanism 32 is a ball screw mechanism. Specifically, the direct-acting mechanism 32 includes a shaft member and a slider (not illustrated in the drawings); when the shaft member is rotated forward using the motor 33, the slider moves along the shaft member and pushes down the piston 31b. Thus, the operating fluid is supplied to the cylinder chamber 12d, the rod 12b is extended, and the pair of gripping portions 11a, 11a is closed. On the other hand, when the motor 33 is driven to rotate the shaft member backward, the piston 31b is pulled up, and the operating fluid is removed from the cylinder chamber 12d. Accordingly, the biasing force of the compression coil spring 24 retracts the rod 12b, making it possible to open the pair of gripping portions 11a, 11a.

In order to control the operation of the driving device 13 configured as described above, the control device 3 is connected to the driving device 13. The control device 3, which is connected to the motor 33, can adjust the degree of opening and the gripping force of the gripping portions 11a, 11a by controlling the operation (such as the direction of rotation, the torque, and the angle of rotation) of the motor 33.

Furthermore, the clip applier 1 includes a shaft 15 in order to connect the driving device 13 and the opening/closing driving mechanism 12 located at the tip end of the clip applier 1, and the tube 25 is disposed in the shaft 15. The tube 25 and the shaft 15 are both flexible, making it possible to change the direction and attitude of the pair of jaws 11, 11 by bending the shaft 15 in various directions such as upward, downward, leftward, and rightward directions or rotating the shaft 15 about the axial line thereof, for example. Furthermore, the driving device 13 includes a direction changing device 34 in order to control the bending motion and the rotating motion, and the direction changing device 34 bends the shaft 15 in various directions or rotates the shaft 15 by a wire or the like, for example. The direction changing device 34 having such a function is also connected to the control device 3, and the operation of the direction changing device 34 is controlled by the control device 3.

### <Operation of Clip Applier>

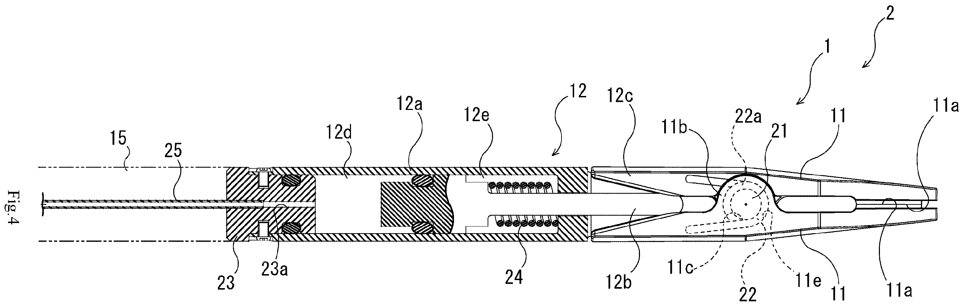

In the clip applier 1 configured as described above, a clip in the approximate V-shape not illustrated in the drawings is loaded between the pair of gripping portions 11a, 11a that are open, as illustrated in Fig. 1. After loading, when the control device 3 drives the motor 33 and the operating fluid is supplied from the pump 31 to the opening/closing driving mechanism 12, the rod 12b is pushed by the operating fluid and is thus extended. As a result of the extension, the base end of the pair of jaws 11, 11 is pushed and opened by the tip of the rod 12b. Such an opening motion of the base end of the pair of jaws 11, 11 leads to closure of the pair of gripping portions 11a, 11a as illustrated in Fig. 4, resulting in squeezing of the clip. In other words, the blood vessel, etc., is ligated.

Contrarily, in the state where the pair of gripping portions 11a, 11a is closed, when the control device 3 reversely rotates the motor 33 and the operating fluid is removed from the opening/closing driving mechanism 12 into the pump 31, the rod 12b that has been pushed by the compression coil spring 24 is retracted. Accordingly, the torsion coil spring 22 causes the pair of gripping portions 11a, 11a to be gradually opened, enabling the pair of gripping portions 11a, 11a to eventually separate from the clip. Thereafter, when the rod 12b is retracted into the vicinity of the lid body 23, the opening motion for opening the tip end of the pair of jaws 11, 11 stops (refer to Fig. 2).

The clip applier 1 configured as described above uses the operating fluid to close the pair of gripping portions 11a, 11a and thus can efficiently transmit force. Therefore, as compared to a conventional clip applier of the wire drive type, the gripping force can be made great; it is possible to squeeze even a clip that requires great gripping force to squeeze, such as a metal clip. Furthermore, in the clip applier 1, the flexible tube 25 can be used for the tube 25 which supplies the operating fluid, and thus it is not always necessary to use the shaft 15 that is rigid. This means that the shaft 15 can be bent in various directions, allowing the pair of jaws 11, 11 better access to an affected area. Note that the shaft 15 does not necessarily need to be flexible and may be a metal pipe or the like which is very rigid.

Furthermore, in the clip applier 1, the compression coil spring 24 is provided in order to retract the rod 12b in the opening/closing driving mechanism 12. Therefore, a single-acting cylinder mechanism can be used for the cylinder mechanism. Accordingly, the structure of the clip applier 1 can be simplified, and the number of components can be reduced. Thus, it is possible to reduce cost for the clip applier 1. Note that the cylinder mechanism used in the clip applier 1 is not always limited to the single-acting cylinder mechanism and may be a double-acting cylinder mechanism. In this case, a tube different from the tube 25 is connected to the spring housing space 12c, making it possible to retract the rod 12b and open the tip end of the pair of jaws 11, 11 by supplying the operating fluid to the spring housing space 12e via said different tube.

### Embodiment 2

A clip applier 1A according to Embodiment 2 is similar in configuration to the clip applier 1 according to Embodiment 1. Therefore, the configuration of the clip applier 1A according to Embodiment 2 will be described focusing on differences from the clip applier 1 according to Embodiment 1; elements that are the same as those of the clip applier 1 according to Embodiment 1 share the same reference signs, and as such, description of the elements will be omitted.

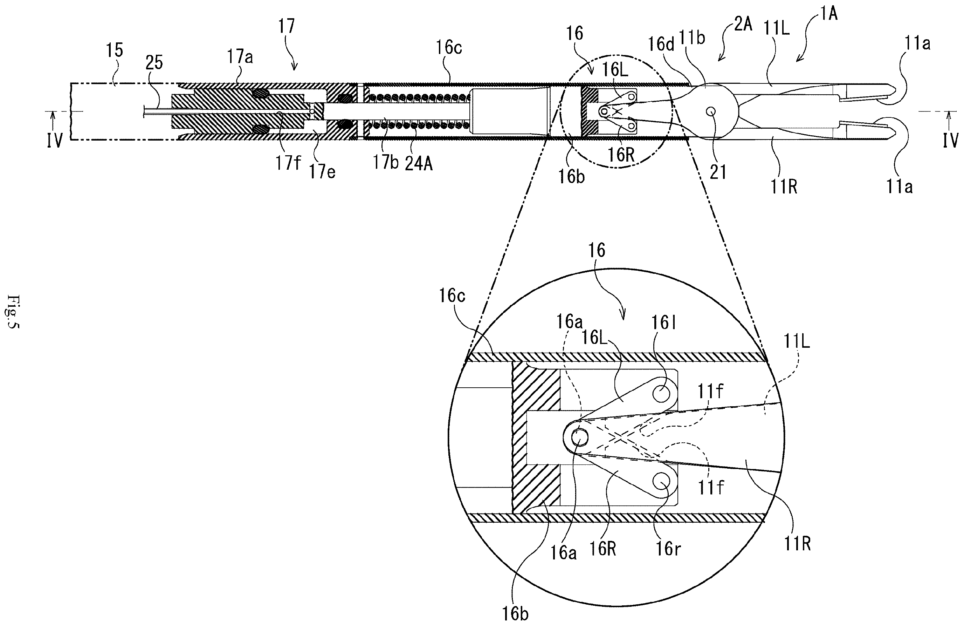

The clip applier 1A according to Embodiment 2 is included in a clip applier system 2A and includes a pair of jaws 11L, 11R, an opening/closing mechanism 16, a driving mechanism 17, and a driving device 13 (refer to Fig. 1), as illustrated in Fig. 5. The pair of jaws 11L, 11R are elongated members and are arranged so that side surfaces thereof at the tip ends on which the gripping portions 11a, 11a are formed face each other. Furthermore, the pair of jaws 11L, 11L includes coupling portions 11b, 11b in the middle in the longitudinal direction. The coupling portions 11b, 11b are each formed in the approximate shape of a circle in plan view, and the pair of jaws 11L, 11R are arranged so as to overlap each other. The pin member 21 is inserted through the coupling portions 11b, 11b overlapping in this manner and rotatably fastens the coupling portions 11b, 11b together. Specifically, the pair of jaws 11L, 11R are also rotated about the pin member 21, allowing opening and closing of the pair of gripping portions 11a, 11a.

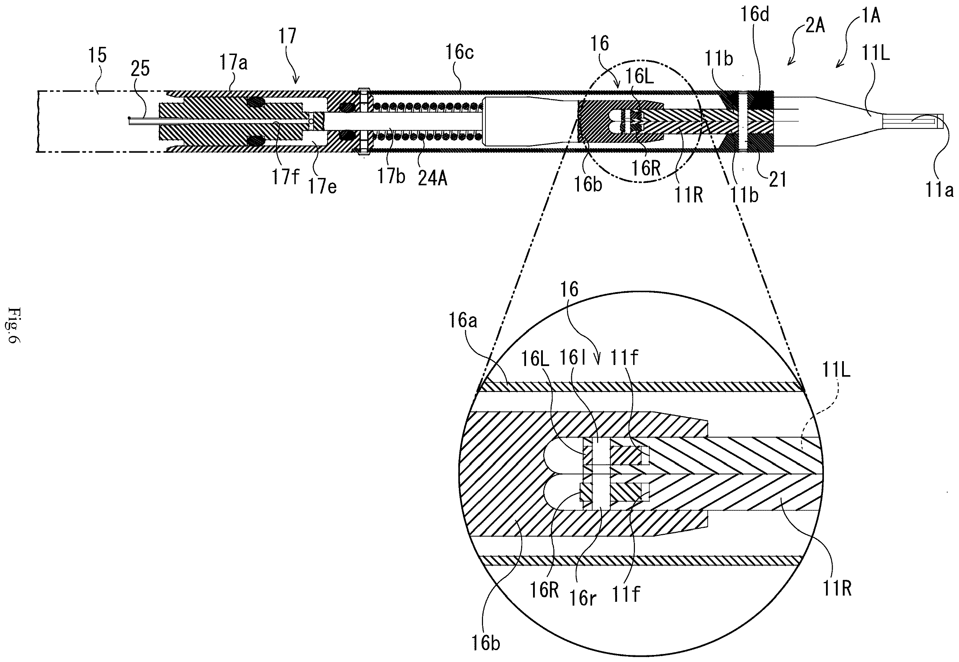

Base end portions of the pair of jaws 11L, 11R are also formed into the same shape, as with the coupling portions 11b, 11b, and are arranged so as to overlap each other in plan view with the tip end of the pair of jaws 11L, 11R open. The base end portions of the pair of jaws 11L, 11R are formed in the approximate shape of a plate and are formed into the approximate U-shape in side view. Specifically, the base end portions of the pair of jaws 11L, 11R include grooves 11f, 11f which penetrate the base end portions in the width directions thereof and are open at the base ends (refer also to Fig. 6), and ends of link members 16L, 16R included in the opening/closing mechanism 16 are inserted through the grooves 11f, 11f.

Each of the pair of link members 16L, 16R is a plate member in the approximate shape of a strip, and one end portion thereof is inserted into a corresponding one of the grooves 11f, 11f of the jaws 11L, 11R. The link members 16L, 16R are fastened to the respective base end portions of the corresponding jaws 11L, 11R by the pin member 16a and are rotatably attached to the corresponding jaws 11L, 11R. The pair of link members 16L, 16R attached in this manner extend diagonally from the base end portions of the jaws 11L, 11R toward the tip end and are arranged in the approximate V-shape in plan view. The other end portion of each of the pair of link members 16L, 16R arranged as just described is coupled to a slider member 16b.

The slider member 16b constitutes the opening/closing mechanism 16 together with the pair of link members 16L, 16R and is housed in a casing 16c. The casing 16c is formed in the approximate shape of a circular pipe in which the slider member 16b is housed in such a manner as to be movable along the axis thereof (in other words, toward the proximal end and toward the distal end). Furthermore, the base end portions of the pair of jaws 11L, 11R are inserted into the tip end of the casing 16c, and the casing 16c includes a pair of attachment pieces 16d, 16d at the tip end. The pair of attachment pieces 16d, 16d are each a member in the approximate shape of a plate and are arranged so that one surface of one attachment piece 16d and one surface of the other attachment piece 16d face each other. The coupling portions 11b, 11b of the pair of jaws 11L, 11R are placed between the pair of attachment pieces 16d, 16d arranged as just described and are rotatably fastened together by the pin member 21.

Meanwhile, a tip end portion of the slider member 16b is formed in the approximate U-shape in side view, as illustrated in Fig. 6, and includes, at the tip end, a groove penetrating the slider member 16b. The base end portions of the pair of jaws 11L, 11R are inserted through this groove. The pair of link members 16L, 16R are inserted through the base end portions of the pair of jaws 11L, 11R, and the tip ends of the pair of link members 16L, 16R are rotatably fastened to the slider member 16b by pin members 16r, 161. Therefore, according to the movement of the slider member 16b, the pair of link members 16L, 16R moves as follows.

Specifically, one of the pair of link members 16L, 16R, i.e., the link member 16L, couples the slider member 16b and the base end portion of one jaw 11L, and when the slider member 16b moves toward the proximal end, the link member 16L moves the base end portion of the one jaw 11L away from one pin member 161. The other link member 16R couples the slider member 16b and the base end portion of the other jaw 11R, and when the slider member 16b moves toward the proximal end, the other link member 16R moves the base end portion of the other jaw 11R away from the other pin member 16r. Thus, the base end of the pair of jaws 11L, 11R is opened, and the pair of gripping portions 11a, 11a is closed accordingly. On the other hand, as the slider member 16b is moved toward the distal end and returned to the original position, the base end of the pair of jaws 11L, 11R is closed, and the pair of gripping portions 11a, 11a is opened. In this manner, the opening/closing mechanism 16 moves the slider member 16b to move the link members 16L, 16R and can thereby open and close the pair of gripping portions 11a, 11a. Furthermore, a driving mechanism 17 is provided on the casing 16c in order to move the slider member 16b.

The driving mechanism 17 is what is called a cylinder mechanism and includes a cylinder 17a and a rod 17b. The cylinder 17a is formed in the approximate shape of a pipe with a closed end and has a bottom at the tip end. The cylinder 17a having such a shape is attached to the base end portion of the casing 16c by the tip end of the cylinder 17a fitting thereto. Furthermore, the rod 17b is housed in the cylinder 17a in such a manner that the rod 17b can be extended and retracted in order to move the slider member 16b. A tip end portion of the rod 17b in the state of being sealed penetrates the tip end of the cylinder 17a and protrudes from the tip end of the cylinder 17a into the casing 16c. A tip end of the rod 17b is coupled to the slider member 16b, and the slider member 16b moves toward the proximal end and toward the distal end in conjunction with the movement of the rod 17b.

The outer diameter of a base end portion of the rod 17b configured as described above substantially matches the inner diameter of the cylinder 17a, and the base end portion of the rod 17b in the state of being sealed slidably fits into the cylinder 17a. The base end portion of the rod 17b is positioned at a distance from the bottom of the cylinder 17a on the distal side; in the cylinder 17a, a cylinder chamber 17e is formed between the base end portion of the rod 17b and the bottom of the cylinder 17a, and the operating fluid is supplied to the cylinder chamber 17e.

Specifically, the tube 25 is connected to the base end portion of the rod 17b, and a communication passage 17f is formed in the rod 17b so as to connect the tube 25 and the cylinder chamber 17e. Thus, the operating fluid supplied from the driving device 13 via the tube 25 can be guided to the cylinder chamber 17e via the communication passage 17f. The base end portion of the rod 17b receives, from the operating fluid in the cylinder chamber 17e, force acting toward the proximal end, that is, force acting in a direction in which the rod 17b is retracted; when the operating fluid is supplied to the cylinder chamber 17e, the rod 17b is retracted. Accordingly, the slider member 16b moves toward the proximal end, and the pair of gripping portions 11a, 11a is closed. Furthermore, the driving mechanism 17 includes a compression coil spring 24A in order to extend the rod 17b.

The compression coil spring 24A is attached to the exterior of a portion of the rod 17b that protrudes from the cylinder 17a, and has one end abutting the slider member 16b and the other end abutting the tip end of the cylinder 17a. Thus, the biasing force against the pressure of the operating fluid in the cylinder chamber 17e is provided by the compression coil spring 24A to the rod 17b via the slider member 16b. Therefore, when the operating fluid is removed from the cylinder chamber 17e using the driving device 13, the rod 17b is extended with assistance of the biasing force of the compression coil spring 24A, and the slider member 16b moves toward the distal end. Accordingly, the pair of gripping portions 11a, 11a is opened.

In this manner, the driving mechanism 17 can extend and retract the rod 17b by supplying and removing the operating fluid, move the slider member 16b, and open and close the tip end of the pair of jaws 11L, 11R. Furthermore, the shaft 15 is provided at the opening end of the cylinder 17a of the driving mechanism 17, and the cylinder 17a of the driving mechanism 17 is connected to the driving device 13 via the shaft 15.

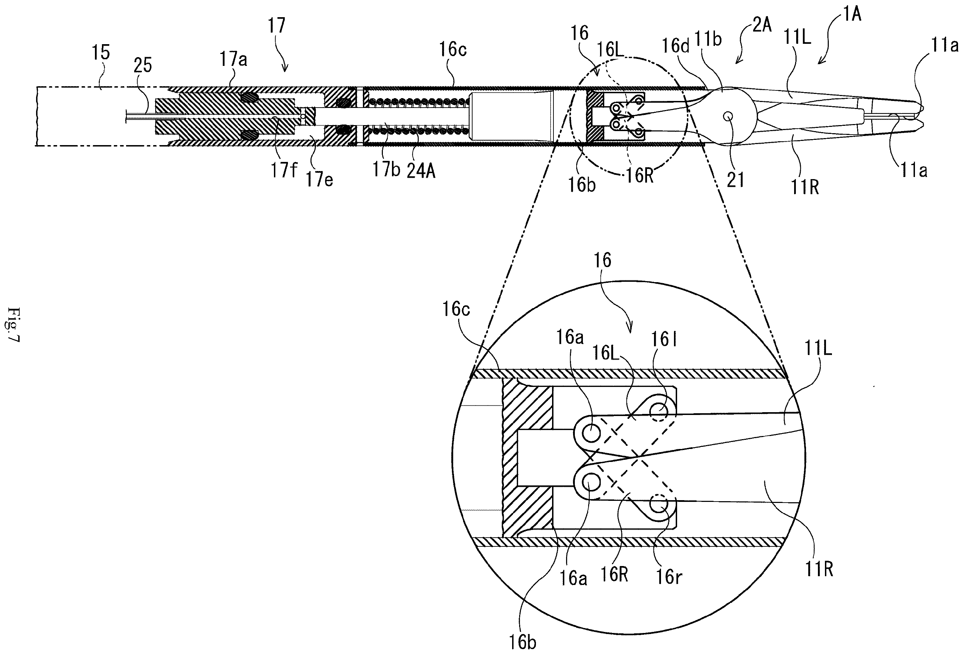

In the clip applier 1A configured as described above, a clip in the approximate V-shape not illustrated in the drawings is loaded between the pair of gripping portions 11a, 11a that are open, as illustrated in Fig. 5. After loading, when the control device 3 drives the motor 33 and the operating fluid is supplied from the pump 31 to the driving mechanism 17, the rod 17b is retracted by the operating fluid. This retraction causes the slider member 16b to move toward the proximal end, and the pair of link members 16L, 16R operates accordingly so as to push and open the base end of the pair of jaws 11L, 11R. This leads to closure of the pair of gripping portions 11a, 11a as illustrated in Fig. 7, resulting in squeezing of the clip. In other words, the blood vessel, etc., is ligated.

Contrarily, in the state where the pair of gripping portions 11a, 11a is closed, when the control device 3 rotates the motor 33 and the operating fluid is removed from the driving mechanism 17, the rod 17b is extended by the biasing force of the compression coil spring 24A. This causes the slider member 16b to move toward the distal end, and the pair of link members 16L, 16R operates accordingly so as to close the base end of the pair of jaws 11L, 11R. Thus, the pair of gripping portions 11a, 11a is gradually opened, enabling the pair of gripping portions 11a, 11a to eventually separate from the clip. As the pair of gripping portions 11a, 11a is further opened, the base ends of the pair of jaws 11L, 11R eventually overlap in plan view, and the opening motion for opening the pair of gripping portions 11a, 11a stops (refer to Fig. 5).

As with the clip applier 1 according to Embodiment 1, the clip applier 1A configured as described above uses the operating fluid to close the pair of gripping portions 11a, 11a and thus can efficiently transmit force. Therefore, with the clip applier 1A, it is possible to squeeze even a metal clip or the like. Furthermore, as a result of including the opening/closing mechanism 16 and the driving mechanism 17, the clip applier 1A can generate great gripping force even with a structure in which the retraction motion of the rod 17b causes the pair of gripping portion 11a, 11a to be closed.

Furthermore, in the clip applier 1A, the cylinder chamber 17e is formed closer to the distal end than the base end portion of the rod 17b in order to retract the rod 17b, and the tube 25 is connected to the base end (that is, the proximal end) of the rod 17b. Regarding this, by forming the communication passage 17f so as to penetrate the rod 17b, it is possible to reduce the increase in the size of the cylinder 17a that is caused by forming, in the cylinder 17a, a passage connecting the tube 25 and the cylinder chamber 17. Thus, the increase in the size of the driving mechanism 17 and the increase in the size of the clip applier 1A can be reduced.

Furthermore, in the clip applier 1A, the compression coil spring 24A is provided in order to extend the rod 17b in the driving mechanism 17. Therefore, a single-acting cylinder mechanism can be used for the cylinder mechanism, and the structure of the clip applier 1A can be simplified. Thus, it is possible to reduce the number of components of the clip applier 1A, reducing cost for the clip applier 1A. Note that as with the opening/closing driving mechanism 12 in the clip applier 1, the cylinder mechanism to be used for the driving mechanism 17 may be a double-acting cylinder mechanism.

### Other Embodiments

The clip applier 1A according to Embodiment 2 is configured to close the tip end of the pair of jaws 11L, 11R by retracting the rod 17b, but does not necessarily need to be configured in this manner. For example, as with the clip applier 1 according to Embodiment 1, the clip applier 1A may be configured so as to close the tip end of the pair of jaws 11L, 11R by extending the rod 17b. In other words, a cylinder mechanism having the same structure as the cylinder mechanism used in the opening/closing driving mechanism 12 according to Embodiment 1 may be used for the driving mechanism 17. Furthermore, the pair of link member 16L, 16R and the base end portions of the pair of jaws 11L, 11R are coupled so that as the slider member 16b moves toward the distal end, the base end of the pair of jaws 11L, 11R is opened. This allows the tip end of the pair of jaws 11L, 11R to be closed as the rod 17b is extended.

From the foregoing description, many modifications and other embodiments of the present invention would be obvious to a person having ordinary skill in the art. Therefore, the foregoing description should be interpreted only as an example and is provided for the purpose of teaching the best mode for carrying out the present invention to a person having ordinary skill in the art. Substantial changes in details of the structures and/or functions of the present invention are possible within the spirit of the present invention.

### Reference Signs List

- 1, 1A: clip applier
- 11, 11L, 11R: jaw
- 11a: gripping portion
- 12: opening/closing driving mechanism
- 12a: cylinder
- 12b: rod
- 16: opening/closing mechanism
- 16L, 16R: link member
- 17: driving mechanism
- 17a: cylinder
- 17b: rod
- 17e: cylinder chamber
- 17f: communication passage
- 22: torsion coil spring (first biasing member)
- 23a: communication passage
- 24: compression coil spring (second biasing member)
- 24A: compression coil spring (biasing member)
- 25: tube

## Claims

1. A hydraulically driven clip applier, comprising:
a pair of jaws that respectively include, at a tip end, a pair of gripping portions capable of holding a clip between the gripping portions and are coupled to each other in a manner to allow the pair of the gripping portions to be opened and closed and when a base end of the pair of the jaws is opened, close the pair of the gripping portions; and
an opening/closing driving mechanism that is a cylinder mechanism including a rod and when supplied with an operating fluid, extends the rod, wherein:
base end portions of the pair of the jaws are positioned at a distance from each other; and
the opening/closing driving mechanism is configured to position a tip end of the rod between the base end portions of the pair of the jaws and open the base end of the pair of the jaws by extending the rod.

2. The hydraulically driven clip applier according to claim 1, further comprising:
a first biasing member that biases at least one of the pair of the jaws to operate the pair of the gripping portions in a direction in which the pair of the gripping portions is opened, wherein:
the pair of the jaws is configured to be closed at the base end when the pair of the gripping portions is opened; and
the opening/closing driving mechanism includes a second biasing member that biases the rod against a pressure of the operating fluid to retract the rod.

3. A hydraulically driven clip applier, comprising:
a pair of jaws that respectively include, at a tip end, a pair of gripping portions capable of holding a clip between the gripping portions and are coupled to each other in a manner to allow the pair of the gripping portions to be opened and closed and when a base end of the pair of the jaws is opened, close the pair of the gripping portions;
an opening/closing mechanism coupled to the base end of the pair of the jaws and configured to open and close the base end of the pair of the jaws; and
a driving mechanism that operates a link of the opening/closing mechanism to open the base end of the pair of the jaws, wherein:
the driving mechanism is a cylinder mechanism including a rod and when supplied with an operating fluid, retracts the rod; and
the opening/closing mechanism includes a pair of links coupled to the rod and the base end of the pair of the jaws and is configured to open the base end of the pair of the jaws using the pair of the links when the rod is retracted.

4. The hydraulically driven clip applier according to claim 3, wherein:
the pair of the jaws opens the pair of the gripping portions by closing the base end of the pair of the jaws;
the opening/closing mechanism is configured to close the base end of the pair of the jaws using the pair of the links when the rod is extended; and
the driving mechanism further includes a biasing member that biases the rod against a pressure of the operating fluid to extend the rod.

5. The hydraulically driven clip applier according to claim 3 or 4, wherein:
the cylinder mechanism includes a cylinder through which the rod protruding from a tip end of the cylinder is inserted in a manner to be retractable;
the cylinder includes, at the tip end, a cylinder chamber to which the operating fluid is supplied to retract the rod; and
a tube through which the operating fluid is supplied is connected to a base end of the rod, and a communication passage is formed in the rod to guide, to the cylinder chamber, the operating fluid supplied through the tube.

6. A hydraulically driven clip applier, comprising:
a pair of jaws that respectively include, at a tip end, a pair of gripping portions capable of holding a clip between the gripping portions and are rotatably coupled to each other in a manner to allow the pair of the gripping portions to be opened along with a closing motion at a base end and allow the pair of the gripping portions to be closed along with an opening motion at the base end;
an opening/closing mechanism coupled to the base end of the pair of the jaws and configured to open and close the base end of the pair of the jaws; and
an opening/closing driving mechanism that, when supplied with an operating fluid, operates the opening/closing mechanism to open the base end of the pair of the jaws, wherein:
the opening/closing driving mechanism is a cylinder mechanism including a rod and when supplied with the operating fluid, operates the rod; and
the opening/closing mechanism includes a pair of links coupled to the rod and the base end of the pair of the jaws and is configured to close the base end of the pair of the jaws using the pair of the links when the rod is operated.
